# EUROPEAN PATENT APPLICATION

(11) **EP 1 602 361 A2**
(43) Date of publication of application: **07.12.2005**
(21) Application number: 05076217.8
(22) Date of filing: 26.05.2005
(51) Int. Cl.: A61K 9/14, A61K 31/545, A61K 31/43

(54) **Antibiotic in the form of a water-soluble powder for veterinary and human use**

(30) Priority: 01.06.2004 BE 200400268
(71) Applicant: Vaccifar BV, 4567 AR Clinge (NL)
(72) Inventor: de Maeyer, Pieter, 2160 Wommelgem (BE)
(74) Representative: Donné, Eddy

(57) **Abstract**

Antibiotic in the form of a water-soluble powder for veterinary and human use, containing at least one unsalted active constituent which is only soluble in water with an alkaline pH and which also contains a salt of a strong base, characterised in that it also contains disodium edetate.

## Description

The present invention concerns an antibiotic in the form of a water-soluble powder for veterinary en human use and a method for preparing a solution containing such an antibiotic.

More specifically, the invention concerns an antibiotic in the form of a water-soluble powder for veterinary and human use, comprising at least one non-salted active constituent which is only soluble in water with an alkaline pH and which also comprises a salt of a strong base.

Such an antibiotic can be used for example for treating cattle or poultry having an infection caused by bacteria which are sensitive to the above-mentioned active constituent.

The solubility in water of such a non-salted active constituent which is only soluble in water with an alkaline pH is limited, and the dissolving process is relatively slow. Moreover, the stability of such an active constituent in water is limited as well.

Both the above-mentioned factors depend strongly on the degree of acidity of the solution and on the presence of metal ions in the water.

Metal ions which are present in the water act as a catalyst and accelerate the decomposition of the above-mentioned active constituent, as a result of which the active working of the solution decreases relatively fast, and the storage life will hence be limited.

In order to obtain a good and fast solubility and a large stability of the active constituent in water, the present invention concerns an antibiotic in the form of a water-soluble powder for veterinary and human use, containing at least one non-salted active constituent which is only soluble in water with an alkaline pH and which also contains a salt of a strong base, whereby it is also provided with disodium edetate.

The above-mentioned unsalted active constituent is for example cephalosporin or amoxicillin, more particularly in the form of amoxicillin trihydrate. The concentration of active constituent is preferably situated between 1 and 85 percent, based on the total weight of the water-soluble powder.

The above-mentioned salt of a strong base preferably contains carbonate, bicarbonate or sulphate ions as an anion and sodium, potassium or ammonium ions as a cation.

The salt provides for an appropriate degree of acidity of the solution of the above-mentioned active constituent in water, since, when it is solved in water, it has a basic character and thus produces a relatively high pH-value. This salt molecule also protects the active constituent against decomposition, which occurs with high as well as with low pH-values.

An example of such a salt of a strong base is sodium carbonate monohydrate.

The disodium edetate (EDTA) is able to bind metal ions such as calcium, copper, nickel, iron, magnesium and manganese, such that these bound metal ions can no longer function as a catalyst for the decomposition reaction of the unsalted active constituent.

In this manner is thus made sure that the above-mentioned unsalted active constituent is more stable in the water and thus remains active for a considerably longer time.

The present invention also concerns a solution which contains an antibiotic in the form of a water-soluble powder, as described above.

The present invention also concerns a pharmaceutical composition for veterinary or human use, which composition is suitable for oral administration and which composition contains an antibiotic as described above, combined with at least one pharmaceutically acceptable excipient.

The present invention also concerns a method for preparing a solution as described above, whereby this method comprises the following steps:
(i) preparing an antibiotic in powder form which contains a combination of the following powdered constituents: at least one unsalted active constituent which is only soluble in water with an alkaline pH, at least one salt of a strong base and disodium edetate; and
(ii) solving the above-mentioned antibiotic in an amount of water in view of its administration to man or animal.

In a special embodiment of the method according to the invention, step (ii) is carried out in two steps, namely preparing a concentrated solution and diluting the concentrated solution.

In order to better explain the characteristics of the invention, the following test results are represented, as an example only without being limitative in any way, which compare different ratios between the constituents of an antibiotic in the form of a water-soluble powder according to the invention.

### TEST 1.

According to a first part of this test, the solubility of amoxicillin trihydrate, as a non-salted active constituent which is only soluble in water with an alkaline pH, was examined.

Several ratios of amoxicillin trihydrate, disodium edetate and, as a salt, sodium carbonate monohydrate were weighed and solved in HPLC water, i.e. "high pressure liquid chromatography water", which consists of demineralised and de-ionised water, more specifically the following weight ratios:

| Amoxicillin trihydrate | Disodium edetate | Sodium carbonate monohydrate |
|---|---|---|
| 5 | 1 | 4 |
| 5 | 1 | 3 |
| 5 | 1 | 2 |
| 5 | 1 | 1 |

The solubility was initially tested by adding the different constituents in steps, whereby they were each time added in the same ratio in relation to each other.

In this manner, it appears to be possible to obtain a 40% solution of amoxicillin trihydrate in water, when the largest amount of sodium carbonate monohydrate is used, more specifically in a ratio of 5/1/4.

Next, the solubility of amoxicillin trihydrate was tested by adding the different constituents once only according to each time one of the preceding ratios.

In this manner, it appears to be possible to obtain a maximum concentration of 20% amoxicillin trihydrate in HPLC water, with ratios of 5/1/4 and 5/1/3.

The solving was done in this case within a shorter and acceptable interval, whereby the solution process took longer with the ratio 5/1/3 than with the ratio 5/1/4.

With the smaller amounts of sodium carbonate monohydrate, namely with the ratios 5/1/2 and 5/1/1, the solving took very long, or a complete solution was not even obtained.

From what precedes, we may conclude that the presence of sodium carbonate monohydrate has a positive influence on the solubility and the speed of the solving process of amoxicillin trihydrate.

In a second part of the test concerned, the stability for 10% and 20% amoxicillin trihydrate solutions in water was examined.

Said study proves that the decomposition of the amoxicillin trihydrate with such relatively large concentrations is too fast for a successful practical use.

It is already known that the decomposition of amoxicillin trihydrate in concentrated solutions and under standard circumstances usually produces amoxicillin dimer.

Since dimerisation is supposed to be the most important decomposition reaction within a concentrated amoxicillin trihydrate solution and under standard circumstances, it was tested whether the more diluted solutions would be more stable.

This was tested by means of amoxicillin trihydrate concentrations of 1% and of 2%, whereby the ratio in relation to the disodium edetate and the sodium carbonate monohydrate, was 5/1/4 respectively. In this experiment, only the amoxicillin trihydrate was analysed and quantified.

When the stability is defined as being the relative active amount of amoxicillin trihydrate of more than 90% in relation to the initial amount, it appears that a 2% amoxicillin trihydrate solution is stable for 3 hours and a 1% solution for 5 hours.

The numerical data from the preceding test are set out in the following table, whereby the represented values are a measure for the stability of amoxicillin trihydrate (AMX).

| Time(h) | 10% AMX | 10%' AMX | 2% AMX | 1% AMX |
|---|---|---|---|---|
| 0 | 100 | 100 | 100 | 100 |
| 3 | - | - | 91 | 93 |
| 4 | 53 | 60 | - | - |
| 5 | - | - | 89 | 91 |
| 7 | 40 | 50 | - | |
| 24 | - | - | 65 | 75 |
| :no value determined | | | | |

It appears from the table that the higher concentrations of amoxicillin trihydrate (AMX) are less stable and thus decompose faster.

### TEST 2.

This test has more or less the same course as the preceding test. Initially, the stability of the amoxicillin trihydrate in combination with sodium carbonate monohydrate and disodium edetate was examined.

At first, different ratios between disodium edetate and amoxicillin trihydrate were used in order to stabilize amoxicillin trihydrate, after which the stability was each time examined for various concentrations of amoxicillin trihydrate in water.

The stability of amoxicillin trihydrate in a mixture of sodium carbonate monohydrate and disodium edetate was tested with different concentrations of sodium edetate in HPLC water and in tap water.

These measurements also show that the higher concentrations of amoxicillin trihydrate decompose at a higher speed.

The stability of a 5% concentration and of a 0.2% concentration of amoxicillin trihydrate in tap water was tested by means of four independent samples, whereby the 5% concentration was kept in a laboratory, at a constant temperature of 20°C and in a dark environment. After two hours, the amoxicillin trihydrate was decomposed to 85.01 ± 6.64% of the initial amount.

The 0.2% concentration was kept in a chicken farm, at a variable temperature, between 21 and 24°C and in a light environment. In this case, the amoxicillin trihydrate was only decomposed to 93.06 ± 5.44% of the initial amount after two hours.

From what precedes we may again conclude that the higher concentrations of amoxicillin trihydrate decompose faster than the lower concentrations.

Further, the homogeneity was examined, from which it appeared that an increasing concentration of sodium edetate makes the homogeneity of the mixture increase. It appears that with a concentration of 5% sodium edetate or more, the homogeneity is acceptable.

An antibiotic in the form of a water-soluble powder for veterinary and human use according to the invention may additionally contain one or several of the following powdered constituents: anti-caking agents, flavourings, aromatic substances or fillers.

In a preferred embodiment of an antibiotic in the form of a water-soluble powder according to the invention, amoxicillin is used as an active constituent.

It is clear that an antibiotic according to the invention may also contain the active constituent clavulane acid.

The present invention is by no means restricted to the concentrations and ratios given as an example; on the contrary, such an antibiotic according to the invention can be made in many ratios and concentrations while still remaining within the scope of the invention.

## Claims

1. Antibiotic in the form of a water-soluble powder for veterinary and human use, containing at least one unsalted active constituent which is only soluble in water with an alkaline pH and which also contains a salt of a strong base, **characterised in that** it also contains disodium edetate.

2. Antibiotic according to claim 1, **characterised in that** the above-mentioned salt of a strong base contains carbonate, bicarbonate or sulphate ions as an anion, and sodium, potassium or ammonium ions as a cation.

3. Antibiotic according to claim 1, **characterised in that** it additionally contains the active constituent clavulane acid.

4. Antibiotic according to claim 1, **characterised in that** it additionally contains one or several of the following constituents in powder form: anti-caking agents, flavourings, aromatic substances or fillers.

5. Antibiotic according to claim 1, **characterised in that** the concentration of the active constituent is situated between 1 and 85 percent, based on the total weight of the water-soluble powder.

6. Solution containing an antibiotic in the form of a water-soluble powder according to one of the preceding claims.

7. Method for preparing a solution according to claim 6, **characterised in that** it comprises the following steps:
(i) preparing an antibiotic in powder form which contains a combination of the following powdered constituents: at least one unsalted active constituent which is only soluble in water with an alkaline pH, at least one salt of a strong base and disodium edetate; and
(ii) solving the above-mentioned antibiotic in an amount of water in view of its administration to man or animal.

8. Method according to claim 7, **characterised in that** step (ii) can be realised in two steps, namely preparing a concentrated solution and diluting the concentrated solution.

9. Pharmaceutical composition for veterinary or human use, which composition is suitable for oral administration, **characterised in that** it contains an antibiotic according to claim 1, combined with at least one pharmaceutically acceptable excipient.
